# EUROPEAN PATENT SPECIFICATION

(11) **EP 4 627 994 B1**
(45) Date of publication and mention of the grant of the patent: **23.09.2026**
(21) Application number: 24749515.3
(22) Date of filing: 12.01.2024
(51) Int. Cl.: A61B 1/00, A61B 1/018

(54) **CONNECTOR FOR INSERTION PORTION OF ENDOSCOPE AND FIXING STRUCTURE FOR INSERTION PORTION**
VERBINDER FÜR EIN EINFÜHRTEIL EINES ENDOSKOPS UND BEFESTIGUNGSSTRUKTUR FÜR EIN EINFÜHRTEIL
CONNECTEUR POUR PARTIE D'INSERTION D'ENDOSCOPE ET STRUCTURE DE FIXATION POUR PARTIE D'INSERTION

(30) Priority: 31.01.2023 CN 202310048582
(43) Date of publication of application: 08.10.2025
(73) Proprietor: Hunan Vathin Medical Instrument Co., Ltd., Xiangtan, Hunan 411100 (CN)
(72) Inventor: ZHOU, Zhenhua, Xiangtan, Hunan 411100 (CN)
(74) Representative: Bayramoglu et al.
(86) International application number: PCT/CN2024/071934
(87) International publication number: WO 2024/160033

(56) References cited:
- WO-A1-2022/128689
- CN-A- 111 920 365
- CN-A- 114 947 698
- CN-A- 114 947 702
- CN-A- 115 998 223
- CN-U- 217 659 776
- US-A1- 2009 318 763
- US-A1- 2013 274 550

## Description

### TECHNICAL FIELD

The present disclosure belongs to the technical field of endoscopy, and in particular relates to a connector for an inserting unit of an endoscope and a fixing structure of the inserting unit.

### BACKGROUND TECHNOLOGY

The inserting unit of an endoscope with at least illumination and imaging functions advances into a human body through a natural orifice or a surgical incision to detect the human body's cavity environment. The bending angle of the active bending section located at the front end of the inserting unit of the endoscope is adjusted through a handle located outside the human body, such that the active bending section is deflected in a preset direction to provide a large visual angle for observation.

The inserting unit of the endoscope includes the active bending section at the front end and the passive bending section at the rear end. Existing inserting units include two structural types. In one structural type, the active bending section and the passive bending section are integrally formed. In the other structural type, the active bending section and the passive bending section are separately manufactured. When the integrally formed inserting unit structure is fixed to the handle, the end of the inserting unit is entirely enclosed and secured by a fixing structure to ensure connection stability of the inserting unit. When the instrument channel of the inserting unit is connected to a corresponding device, a short connection tube is provided between the instrument channel and the corresponding device to prevent interference with other channels in the inserting unit, such as the wiring channel and the traction wire channel. The short connection tube can effectively avoid the influence on the wiring channel and the traction wire channel, but in practical applications, to cooperate with the internal space of the endoscope handle, the short connection tube is typically a soft tube, which increases the operational difficulty of the endoscope. WO2022/128689A1, which discloses the features of the preamble of claim 1, discloses an endoscope comprising: a distal tip unit configured to be inserted into a patient's body cavity; a proximal endoscope handle; an insertion tube and a bending section, the insertion tube and the bending section connecting the endoscope handle and the distal tip unit; a working channel provided in the insertion tube and the bending section and extending from the endoscope handle towards the distal tip unit; and a connector adapted to be attachable to the endoscope handle; wherein the connector comprises a first channel having a first inlet opening and a second channel having a second inlet opening, wherein the second channel joins with the first channel, so that the connector has an outlet opening being in connection with the first inlet opening and the second inlet opening; wherein the outlet opening, when the connector is attached to the endoscope, is in connection with the working channel of the endoscope; and wherein the connector is a multi-piece connector comprising at least a first part and a separate second part that are attached to each other.

### CONTENT OF THE INVENTION

To solve the above technical problems, the present disclosure provides a connector for an inserting unit of an endoscope and a fixing structure of the inserting unit. The invention is defined in independent claim 1, with the dependent claims defining preferred embodiments thereof.

The present disclosure is implemented as follows:
A connector for an inserting unit of an endoscope includes a fixing unit and a mounting unit, where an operation channel is provided inside the fixing unit, and the operation channel is provided with a first opening located on a surface of the fixing unit; one end of the mounting unit is fixed to the fixing unit, and the other end of the mounting unit is configured to be connected to an inserting unit; the inserting unit is provided with an instrument channel, a traction wire channel, and a wiring channel; and when the mounting unit is connected to the inserting unit, the first opening communicates with the instrument channel, and the traction wire channel and the wiring channel communicate with an environment outside the fixing unit.

In the above technical solution, when the connector fixes the inserting unit, the instrument channel of the inserting unit is separated from other channels, allowing a relevant device to directly enter the instrument channel through the operation channel and the first opening for procedures. This design eliminates the need for a separate connection tube between the operation channel and the instrument channel, reducing the operational difficulty of the endoscope. Additionally, omitting the connection tube improves the responsiveness of operations involving the instrument channel.

The end of the mounting unit connected to the inserting unit is provided with a receiving cavity; the receiving cavity communicates with the first opening; and the receiving cavity is sleeved outside the inserting unit. The design improves fixation stability of the inserting unit.

The receiving cavity is provided with two open ends along an axial direction; a side wall of the receiving cavity is provided with a first gap; and two ends of the first gap communicate with the two open ends, respectively. The presence of the first gap provides a range of variability in the inner size of the receiving cavity, facilitating the mounting of the inserting unit and stabilizing its fixation position.

Furthermore, preferably, a length direction of the first gap is parallel to the axial direction of the receiving cavity; alternatively, an arc length of the first gap along a radial direction of the receiving cavity is defined as a width of the first gap; and a central angle corresponding to the width of the first gap is less than 180°. The width of the first gap should not be excessively large to prevent the inserting unit from falling out of the receiving cavity.

Furthermore, preferably, the first gap is divided into a constant segment and a diameter-expanding segment along a direction from the receiving cavity to the fixing unit; and a width of the constant segment remains unchanged, and a width of the diameter-expanding segment gradually increases along the direction from the receiving cavity to the fixing unit. After the inserting unit is inserted into the receiving cavity, adhesive fixation is subsequently required. The diameter-expanding segment ensures that the side walls of the receiving cavity on the two sides of the first gap remain sufficiently far from the end of the inserting unit, thereby preventing adhesive overflow from entering the end of the inserting unit and affecting the channel outlet.

Furthermore, preferably, an adaptation surface is provided on an outer side wall of the receiving cavity, and the adaptation surface is a planar structure. The adaptation surface is mainly configured to be attached to an inner side wall of an endoscope handle, thereby avoiding displacement of the connector as a whole.

Furthermore, preferably, the fixing unit is provided with a connection end surface attached to an end surface of the inserting unit, and the first opening is located on the connection end surface; an edge of the connection end surface is provided with a first curved segment and at least one second curved segment; and the first curved segment is configured to be attached to an edge of the wiring channel, and the second curved segment is configured to be attached to an edge of the traction wire channel. The first curved segment and the second curved segment are mainly configured to prevent height differences arising between the outer side wall of the fixing unit and the channel of the inserting unit, thereby facilitating subsequent wiring.

Furthermore, preferably, the operation channel includes a connection segment and an operation segment; One end of the connection segment forms the first opening, and the other end of the connection segment forms a second opening; and the second opening communicates with the operation segment; and a projection surface of the first opening onto a plane where the second opening is located along an axial direction of the connection segment is located within the second opening. The second opening is larger than the first opening to facilitate entry of a relevant device into the instrument channel.

Furthermore, preferably, an intersection line between an inner side wall of the connection segment and a plane including an axis of the connection segment is defined as a first intersection line; the first intersection line is structured as a straight line or a curved line; a distance between the first intersection line and the axis of the connection segment along a direction from the first opening to the second opening gradually increases; an intersection line between an outer side wall of the connection segment and the plane including the axis of the connection segment is defined as a second intersection line; the second intersection line is structured as a straight line or a curved line; and a distance between the second intersection line and the axis of the connection segment along the direction from the first opening to the second opening gradually increases. Both the inner side wall and the outer side wall of the connection segment possess inclined guiding features. The inner side wall assists in guiding a relevant device to smoothly move from the second opening to the first opening, and the outer side wall facilitates layout of related wires from the wiring channel and the traction wire channel.

A fixing structure for an inserting unit of an endoscope includes the connector for the inserting unit of the endoscope according to any one of the above paragraphs and the inserting unit, where an active bending section and a passive bending section of the inserting unit are integrally arranged; the receiving cavity is sleeved on an end of the passive bending section far from the active bending section; and the instrument channel communicates with the first opening. When the inserting unit is fixed, the instrument channel of the inserting unit is separated from other channels to facilitate subsequent operations and usage.

The present disclosure has the following beneficial effects:
1. In the present disclosure, when the connector is configured to fix the inserting unit, the instrument channel of the inserting unit is separated from other channels to allow the instrument channel to directly communicate with an operation channel. The wiring channel and the traction wire channel communicate with the environment outside the connector. The present disclosure ensures mutual independence of multiple channels, and eliminates the need for a short connection tube between the operation channel and the inserting unit, reducing operational difficulty during endoscope use.
2. In the present disclosure, the end of the inserting unit is fixed through the receiving cavity to enhance stability at the fixation point. Additionally, the first gap is formed on the side wall of the receiving cavity to facilitate mounting of the inserting unit into the receiving cavity while ensuring connection stability between the receiving cavity and the inserting unit.
3. In the present disclosure, the adaptation surface is provided on the outer side wall of the receiving cavity to assist mounting and positioning, enabling the outer side wall of the receiving cavity to be better attached to the inner side wall of the endoscope handle. The adaptation surface is a planar structure to prevent rotation of the receiving cavity and improve overall stability of the device.
4. In the present disclosure, the first curved segment attached to the edge of the wiring channel and the second curved segment attached to the edge of the traction wire channel are formed at the edge of the connection end surface. The present disclosure eliminates a height difference between the surface of the fixing unit and the edge of the channel, preventing friction between wires extending from the wiring channel and the traction wire channel at channel edges.
5. In the present disclosure, the second opening of the connection segment is larger than the first opening to facilitate entry of instruments into the connection segment. Additionally, both the inner side wall and the outer side wall of the connection segment are inclined. The inner side wall can guide the relevant device to smoothly move from the second opening to the first opening, and the outer side wall assists in smooth laying of the wires extending from the wiring channel and the traction wire channel.

### DESCRIPTION OF THE DRAWINGS

To describe the technical solutions in the embodiments of the present disclosure more clearly, the following briefly introduces the drawings required for describing the embodiments or the prior art. Apparently, the drawings in the following description show merely some embodiments of the present disclosure, and persons of ordinary skill in the art may still derive other drawings from these drawings without creative efforts.
FIG. 1 is a first overall structural schematic diagram of a connector according to an embodiment of the present disclosure;
FIG. 2 is a second overall structural schematic diagram of the connector according to an embodiment of the present disclosure;
FIG. 3 is a sectional view of the connector according to an embodiment of the present disclosure;
FIG. 4 is a structural diagram of an inserting unit according to an embodiment of the present disclosure;
FIG. 5 is a schematic diagram of the connector and the inserting unit that match with each other according to an embodiment of the present disclosure;
FIG. 6 is a partial structural detail diagram of the connector and the inserting unit that match with each other according to an embodiment of the present disclosure; and
FIG. 7 is a structural diagram of the connector and the inserting unit that are provided in an endoscope handle according to an embodiment of the present disclosure.

### Reference Numerals:

100. fixing unit; 110. operation channel; 111. connection segment; 112. operation segment; 121. first opening; 122. second opening; 131. first curved segment; 132. second curved segment; 200. mounting unit; 210. receiving cavity; 211. adaptation surface; 220. first gap; 221. constant segment; 222. diameter-expanding segment; 300. inserting unit; 310. instrument channel; 320. traction wire channel; 330. wiring channel; and 400. endoscope handle.

### SPECIFIC IMPLEMENTATIONS

The following description provides many different embodiments or examples for implementing different features of the present disclosure. The elements and arrangements described in the following specific examples are only intended to concisely express the present disclosure, and are only for illustration purposes, rather than to limit the present disclosure.

### Embodiment 1

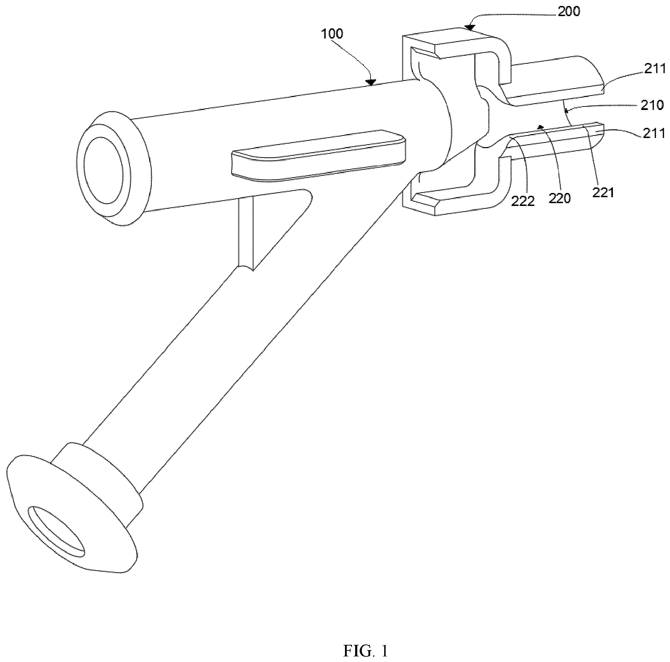

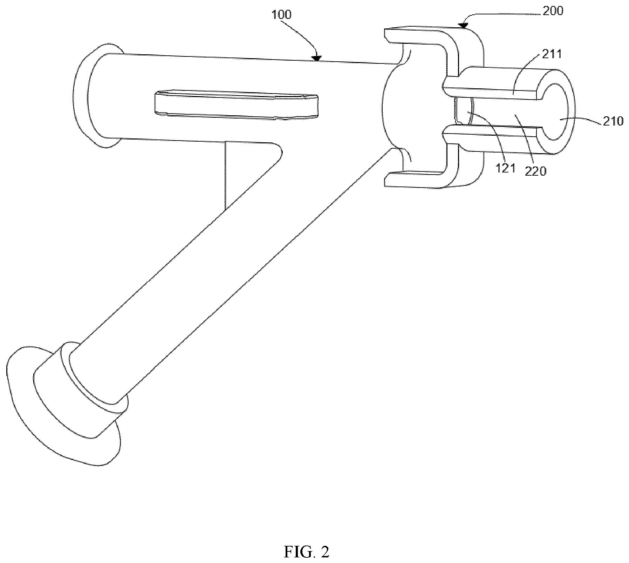

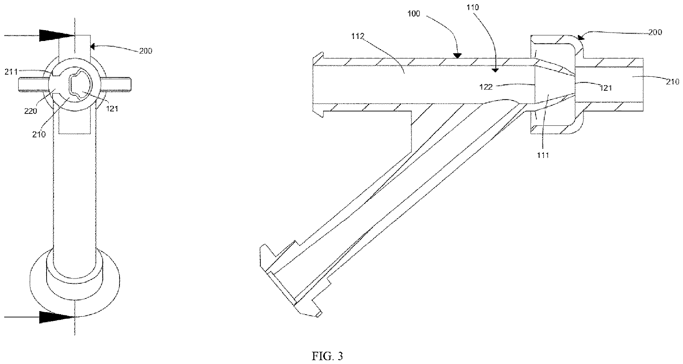

This embodiment provides a connector for an inserting unit of an endoscope. As shown in FIG. 1 to FIG. 7, the connector provided by this embodiment is applicable to integrally formed inserting unit 300 of the endoscope with multiple channels, and specifically includes fixing unit 100 and mounting unit 200. Operation channel 110 is provided inside the fixing unit 100. The operation channel 110 generally has a Y-shaped structure. As shown in FIG. 1 and FIG. 2, the Y-shaped structure includes one end forming first opening 121 located on a surface of the fixing unit 100, one end for use with a negative pressure suction device, and a last end for use with biopsy forceps. One end of the mounting unit 200 is fixed to the fixing unit 100, and the other end of the mounting unit 200 is configured to be connected to the inserting unit 300. The inserting unit 300 is provided with instrument channel 310, traction wire channel 320, and wiring channel 330. When the mounting unit 200 is connected to the inserting unit 300, the first opening 121 communicates with the instrument channel 310, and the traction wire channel 320 and wiring channel 330 communicate with an environment outside the fixing unit 100.

When the connector fixes the inserting unit 300, the instrument channel 310 communicates with the operation channel 110, and the wiring channel 330 and traction wire channel 320 communicate with the external environment, thereby separating the instrument channel 310 from other channels. In prior art, a short connection tube structure is provided between the operation channel 110 and the instrument channel 310. In contrast, the connector structure provided by this embodiment omits the short connection tube, allowing a relevant device to directly enter the instrument channel 310 from the operation channel 110. The design prevents operational difficulty caused by the short connection tube and improves overall usability of the endoscope. Additionally, the omission of the short connection tube enables faster response during operation of the relevant device. Furthermore, when there is a short connection tube provided between the operation channel 110 and the inserting unit 300, the short connection tube will have two connection points that carry risks of leakage and detachment. By omitting the short connection tube, this embodiment avoids risks associated with the connection points and enables a more compact layout within endoscope handle 400.

When the mounting unit 200 is connected to the inserting unit 300, it is sleeved outside the inserting unit 300. The end of the mounting unit 200 connected to the inserting unit 300 is provided with receiving cavity 210. The receiving cavity 210 communicates with the first opening 121. When the receiving cavity 210 is sleeved outside the inserting unit 300, a position of the inserting unit 300 is adjusted to ensure that the instrument channel 310 of the inserting unit 300 communicates with the first opening 121 while the wiring channel 330 and traction wire channel 320 communicate with the external environment.

To improve connection stability between the receiving cavity 210 and the inserting unit 300, an inner diameter of the receiving cavity 210 should not be excessively large. However, if the inner diameter of the receiving cavity 210 is too small, mounting of the inserting unit 300 becomes difficult. To satisfy both requirements of connection stability and ease of assembly, a side wall of the receiving cavity 210 is provided with first gap 220. Two axial ends of the receiving cavity 210 are open ends, with one open end close to the first opening 121 and the other open end far from the first opening 121. The two ends of the first gap 220 communicate with the two open ends, respectively, respectively. The presence of the first gap 220 allows a variable range for an inner size of the receiving cavity 210, facilitating mounting of the inserting unit 300. After side walls on two sides of the first gap 220 are expanded by the inserting unit 300, they exhibit a tendency to close, thereby tightly pressing against an outer surface of the inserting unit 300 and fixing the position of the inserting unit 300.

To facilitate machining, a length direction of the first gap 220 is parallel to an axial direction of the receiving cavity 210. Preferably, an arc length of the first gap 220 along a radial direction of the receiving cavity 210 is defined as a width of the first gap 220. In this embodiment, a central angle corresponding to the width of the first gap 220 is less than 180°. The width of the first gap 220 can vary, but all widths must satisfy the above central angle condition and the width of the first gap 220 should not be excessively large to prevent the inserting unit 300 from falling out of the first gap 220. Generally, the central angle corresponding to the width of the first gap 220 ranges between 60° and 90°.

In this embodiment, the first gap 220 is provided with diameter-expanding segment 222. Along a direction from the receiving cavity 210 to the fixing unit 100, the first gap 220 is sequentially divided into constant segment 221 and the diameter-expanding segment 222. A width of the constant segment 221 remains unchanged, and a width of the diameter-expanding segment 222 gradually increases. Specifically, the width of the diameter-expanding segment 222 gradually increases along the direction from the receiving cavity 210 to the fixing unit 100. When the inserting unit 300 is fixed by the receiving cavity 210, subsequent adhesive bonding is required for reinforcement. To prevent adhesive droplets from falling onto the end of the inserting unit 300, the diameter-expanding segment 222 is provided to ensure side walls of the receiving cavity 210 on both sides of the first gap 220 remain as far away as possible from the end of the inserting unit 300. Specifically, after machining the first gap 220, a chamfering process is directly applied to a position of the first gap 220 close to the end of the inserting unit 300. The first gap 220 formed far from the end of the inserting unit 300 is shown in FIG. 2.

Adaptation surface 211 is provided on an outer side wall of the receiving cavity 210. The adaptation surface 211 is a planar structure. After the connector is provided in the endoscope handle 400, the adaptation surface 211 is attached to an inner side wall of the handle to achieve mounting and positioning. In this embodiment, the adaptation surface 211 can be provided on the two sides of the first gap 220. As shown in FIG. 1 and FIG. 2, the adaptation surface restricts the width of the first gap 220 while providing positioning, thereby preventing changes to the inner diameter of the receiving cavity 210 during use and further enhancing mounting stability of the inserting unit 300.

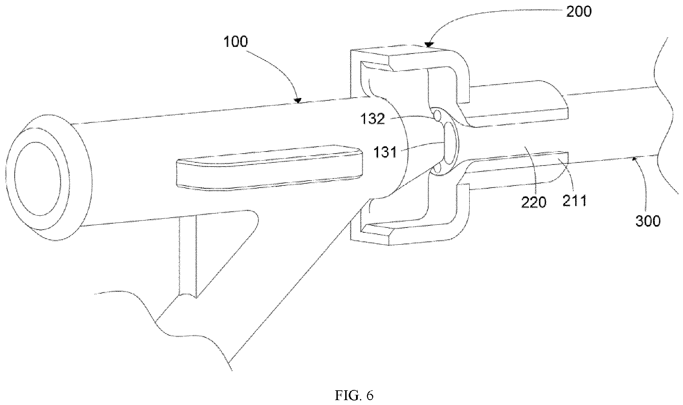

An end surface of the fixing unit 100 attached to an end surface of the inserting unit 300 is a connection end surface. The first opening 121 is located on the connection end surface. An edge of the connection end surface is provided with first curved segment 131 and at least one second curved segment 132. As shown in FIG. 6, the first curved segment 131 is configured to be attached to an edge of the wiring channel 330, and the second curved segment 132 is configured to be attached to an edge of the traction wire channel 320. When the inserting unit 300 is fixed to the fixing unit 100, the first curved segment 131 exhibits no height difference relative to the edge of the wiring channel 330, and the second curved segment 132 exhibits no height difference relative to the edge of the traction wire channel 320. Wires led out from these channels can be directly and normally laid, and will not rub against or be damaged at the edge of the wiring channel 330 during laying.

In this embodiment, the operation channel 110 includes connection segment 111 and operation segment 112. One end of the connection segment 111 forms the first opening 121, and the other end of the connection segment 111 forms second opening 122. The second opening 122 communicates with the operation segment 112. The operation segment 112 includes an end for use with the negative pressure suction device and an end for use with the biopsy forceps. A projection surface of the first opening 121 onto a plane where the second opening 122 is located along an axial direction of the connection segment 111 is located within the second opening 122.

An inner side wall and an outer side wall of the connection segment 111 collectively resemble a side wall structure of a truncated cone, as shown in FIG. 1, FIG. 2, and FIG. 3. However, considering that the first opening 121 must communicate with the instrument channel 310 of the inserting unit 300, to facilitate subsequent use, the shape of the first opening 121 matches a cross-sectional shape of the instrument channel 310. A cross-section of the instrument channel 310 generally exhibits an irregular annular structure. Therefore, a depression or protrusion is formed on the inner side wall of the connection segment 111 close to the first opening 121. Due to the presence of the first curved segment 131 and the second curved segment 132, the outer side wall of the connection segment 111 also includes a depression or protrusion. An intersection line between an inner side wall of the connection segment 111 and a plane including an axis of the connection segment 111 is defined as a first intersection line. The first intersection line is structured as a straight line or a curved line. Along a direction from the first opening 121 to the second opening 122, a distance between the first intersection line and the axis of the connection segment 111 gradually increases. An intersection line between an outer side wall of the connection segment 111 and the plane including the axis of the connection segment 111 is defined as a second intersection line. The second intersection line is structured as a straight line or a curved line. Along the direction from the first opening 121 to the second opening 122, a distance between the second intersection line and the axis of the connection segment 111 gradually increases. Under this configuration, both the inner side wall and the outer side wall of the connection segment 111 possess inclined guiding features. The inner side wall assists in guiding a relevant device to smoothly move from the second opening 122 to the first opening 121, and the outer side wall facilitates layout of related wires from the wiring channel 330 and the traction wire channel 320.

### Embodiment 2

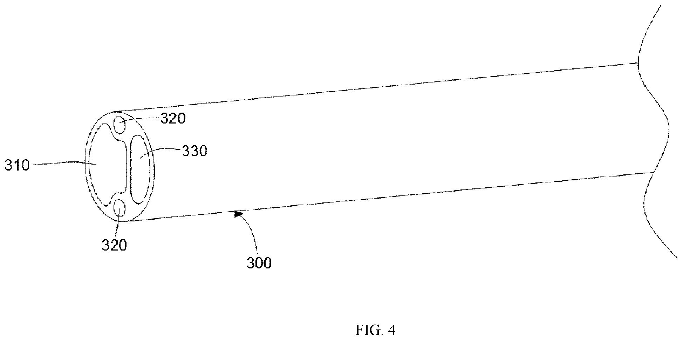

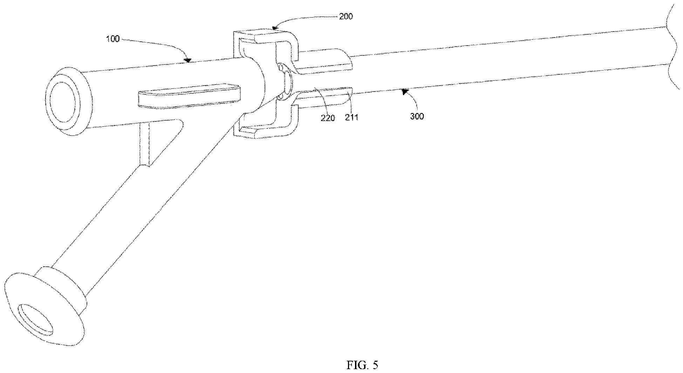

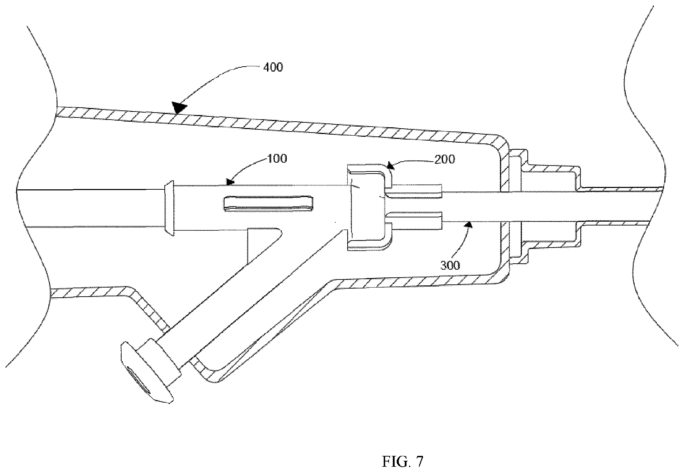

This embodiment provides a fixing structure for an inserting unit of an endoscope. As shown in FIG. 4 to FIG. 7, the fixing structure includes the connector for the inserting unit of the endoscope provided in Embodiment 1 and further includes the inserting unit 300. A structural diagram of the inserting unit 300 is shown in FIG. 4. An active bending section and a passive bending section of the inserting unit 300 are integrally arranged. The receiving cavity 210 is sleeved on an end of the passive bending section far from the active bending section. The instrument channel 310 communicates with the first opening 121, as shown in FIG. 5 and FIG. 6. When the inserting unit 300 is fixed, the instrument channel 310 of the inserting unit 300 is separated from other channels to facilitate subsequent operations and usage. A schematic diagram of the fixing structure provided in the endoscope handle 400 is shown in FIG. 7.

## Claims

1. A connector for an inserting unit of an endoscope, comprising:
a fixing unit (100) and a mounting unit (200), wherein
an operation channel (110) is provided inside the fixing unit (100), and the operation channel (110) is provided with a first opening (121) located on a surface of the fixing unit (100);
one end of the mounting unit (200) is fixed to the fixing unit (100), and the other end of the mounting unit (200) is configured to be connected to an inserting unit (300); and the inserting unit (300) is provided with an instrument channel (310), a traction wire channel (320), and a wiring channel (330); and
when the mounting unit (200) is connected to the inserting unit (300), the first opening (121) communicates with the instrument channel (310), and the traction wire channel (320) and the wiring channel (330) communicate with an environment outside the fixing unit (100);
the end of the mounting unit (200) connected to the inserting unit (300) is provided with a receiving cavity (210); the receiving cavity (210) communicates with the first opening (121);
wherein the receiving cavity (210) is provided with two open ends along an axial direction;
**characterised in that**: the receiving cavity (210) is sleeved outside the inserting unit (300), and a side wall of the receiving cavity (210) is provided with a first gap (220); and two ends of the first gap (220) communicate with the two open ends, respectively.

2. The connector for the inserting unit of the endoscope according to claim 1, wherein
a length direction of the first gap (220) is parallel to the axial direction of the receiving cavity (210); alternatively, an arc length of the first gap (220) along a radial direction of the receiving cavity (210) is defined as a width of the first gap (220); and a central angle corresponding to the width of the first gap (220) is less than 180°.

3. The connector for the inserting unit of the endoscope according to claim 2, wherein
the first gap (220) is divided into a constant segment (221) and a diameter-expanding segment (222) along a direction from the receiving cavity (210) to the fixing unit (100); a width of the constant segment (221) remains unchanged, and a width of the diameter-expanding segment (222) gradually increases along the direction from the receiving cavity (210) to the fixing unit (100).

4. The connector for the inserting unit of the endoscope according to claim 1, wherein
an adaptation surface (211) is provided on an outer side wall of the receiving cavity (210), and the adaptation surface (211) is a planar structure.

5. The connector for the inserting unit of the endoscope according to claim 1, wherein
the fixing unit (100) is provided with a connection end surface attached to an end surface of the inserting unit (300), and the first opening (121) is located on the connection end surface; and
an edge of the connection end surface is provided with a first curved segment (131) and at least one second curved segment (132); and the first curved segment (131) is configured to be attached to an edge of the wiring channel (330), and the second curved segment (132) is configured to be attached to an edge of the traction wire channel (320).

6. The connector for the inserting unit of the endoscope according to claim 1, wherein
the operation channel (110) comprises a connection segment (111) and an operation segment (112); one end of the connection segment (111) forms the first opening (121), and the other end of the connection segment (111) forms a second opening (122); and the second opening (122) communicates with the operation segment (112); and
a projection surface of the first opening (121) onto a plane where the second opening (122) is located along an axial direction of the connection segment (111) is located within the second opening (122).

7. The connector for the inserting unit of the endoscope according to claim 6, wherein
an intersection line between an inner side wall of the connection segment (111) and a plane comprising an axis of the connection segment (111) is defined as a first intersection line; the first intersection line is structured as a straight line or a curved line; and a distance between the first intersection line and the axis of the connection segment (111) along a direction from the first opening (121) to the second opening (122) gradually increases; and
an intersection line between an outer side wall of the connection segment (111) and the plane comprising the axis of the connection segment (111) is defined as a second intersection line; the second intersection line is structured as a straight line or a curved line; and a distance between the second intersection line and the axis of the connection segment (111) along the direction from the first opening (121) to the second opening (122) gradually increases.

8. A fixing structure for an inserting unit of an endoscope, comprising the connector for the inserting unit of the endoscope according to any one of claims 1 to 7 and the inserting unit (300), wherein
an active bending section and a passive bending section of the inserting unit (300) are integrally arranged; the receiving cavity (210) is sleeved on an end of the passive bending section far from the active bending section; and the instrument channel communicates with the first opening (121).

## Patentansprüche

1. Verbinder für eine Einführeinheit eines Endoskops, umfassend:
eine Befestigungseinheit (100) und eine Montageeinheit (200), wobei
ein Arbeitskanal (110) innerhalb der Befestigungseinheit (100) vorgesehen ist und der Arbeitskanal (110) mit einer ersten Öffnung (121) versehen ist, die auf einer Oberfläche der Befestigungseinheit (100) angeordnet ist;
ein Ende der Montageeinheit (200) an der Befestigungseinheit (100) befestigt ist und das andere Ende der Montageeinheit (200) dazu eingerichtet ist, mit einer Einführeinheit (300) verbunden zu werden; und die Einführeinheit (300) mit einem Instrumentenkanal (310), einem Zugdrahtkanal (320) und einem Leitungskanal (330) versehen ist; und
wenn die Montageeinheit (200) mit der Einführeinheit (300) verbunden ist, die erste Öffnung (121) mit dem Instrumentenkanal (310) in Verbindung steht und der Zugdrahtkanal (320) sowie der Leitungskanal (330) mit einer Umgebung außerhalb der Befestigungseinheit (100) in Verbindung stehen;
das mit der Einführeinheit (300) verbundene Ende der Montageeinheit (200) mit einem Aufnahmeraum (210) versehen ist; der Aufnahmeraum (210) mit der ersten Öffnung (121) in Verbindung steht;
wobei der Aufnahmeraum (210) entlang einer Axialrichtung zwei offene Enden aufweist; **dadurch gekennzeichnet, dass** der Aufnahmeraum (210) außen auf die Einführeinheit (300) aufgeschoben ist und eine Seitenwand des Aufnahmeraums (210) mit einem ersten Spalt (220) versehen ist; und die beiden Enden des ersten Spalts (220) jeweils mit den beiden offenen Enden in Verbindung stehen.

2. Verbinder für die Einführeinheit des Endoskops nach Anspruch 1, wobei
eine Längsrichtung des ersten Spalts (220) parallel zur Axialrichtung des Aufnahmeraums (210) verläuft; alternativ eine Bogenlänge des ersten Spalts (220) entlang einer Radialrichtung des Aufnahmeraums (210) als Breite des ersten Spalts (220) definiert ist; und ein der Breite des ersten Spalts (220) entsprechender Mittelpunktswinkel kleiner als 180° ist.

3. Verbinder für die Einführeinheit des Endoskops nach Anspruch 2, wobei
der erste Spalt (220) entlang einer Richtung vom Aufnahmeraum (210) zur Befestigungseinheit (100) in einen Konstantabschnitt (221) und einen Durchmessererweiterungsabschnitt (222) unterteilt ist; eine Breite des Konstantabschnitts (221) unverändert bleibt; und eine Breite des Durchmessererweiterungsabschnitts (222) entlang der Richtung vom Aufnahmeraum (210) zur Befestigungseinheit (100) allmählich zunimmt.

4. Verbinder für die Einführeinheit des Endoskops nach Anspruch 1, wobei
eine Anpassungsfläche (211) an einer äußeren Seitenwand des Aufnahmeraums (210) vorgesehen ist und die Anpassungsfläche (211) eine ebene Struktur ist.

5. Verbinder für die Einführeinheit des Endoskops nach Anspruch 1, wobei die Befestigungseinheit (100) mit einer Verbindungsendfläche versehen ist, die an einer Endfläche der Einführeinheit (300) anliegt, und die erste Öffnung (121) auf der Verbindungsendfläche angeordnet ist; und
ein Rand der Verbindungsendfläche mit einem ersten gekrümmten Abschnitt (131) und wenigstens einem zweiten gekrümmten Abschnitt (132) versehen ist; und der erste gekrümmte Abschnitt (131) dazu eingerichtet ist, an einem Rand des Leitungskanals (330) anzuliegen, und der zweite gekrümmte Abschnitt (132) dazu eingerichtet ist, an einem Rand des Zugdrahtkanals (320) anzuliegen.

6. Verbinder für die Einführeinheit des Endoskops nach Anspruch 1, wobei der Arbeitskanal (110) einen Verbindungsabschnitt (111) und einen Arbeitsabschnitt (112) umfasst; ein Ende des Verbindungsabschnitts (111) die erste Öffnung (121) bildet und das andere Ende des Verbindungsabschnitts (111) eine zweite Öffnung (122) bildet; und die zweite Öffnung (122) mit dem Arbeitsabschnitt (112) in Verbindung steht; und
eine Projektionsfläche der ersten Öffnung (121) auf eine Ebene, in der die zweite Öffnung (122) entlang einer Axialrichtung des Verbindungsabschnitts (111) angeordnet ist, innerhalb der zweiten Öffnung (122) liegt.

7. Verbinder für die Einführeinheit des Endoskops nach Anspruch 6, wobei eine Schnittlinie zwischen einer inneren Seitenwand des Verbindungsabschnitts (111) und einer Ebene, welche eine Achse des Verbindungsabschnitts (111) umfasst, als eine erste Schnittlinie definiert ist; die erste Schnittlinie als eine gerade Linie oder eine gekrümmte Linie ausgebildet ist; und ein Abstand zwischen der ersten Schnittlinie und der Achse des Verbindungsabschnitts (111) entlang einer Richtung von der ersten Öffnung (121) zur zweiten Öffnung (122) allmählich zunimmt; und
eine Schnittlinie zwischen einer äußeren Seitenwand des Verbindungsabschnitts (111) und der Ebene, welche die Achse des Verbindungsabschnitts (111) umfasst, als eine zweite Schnittlinie definiert ist; die zweite Schnittlinie als eine gerade Linie oder eine gekrümmte Linie ausgebildet ist; und ein Abstand zwischen der zweiten Schnittlinie und der Achse des Verbindungsabschnitts (111) entlang der Richtung von der ersten Öffnung (121) zur zweiten Öffnung (122) allmählich zunimmt.

8. Befestigungsstruktur für eine Einführeinheit eines Endoskops, umfassend den Verbinder für die Einführeinheit des Endoskops nach einem der Ansprüche 1 bis 7 sowie die Einführeinheit (300), wobei
ein aktiver Biegeabschnitt und ein passiver Biegeabschnitt der Einführeinheit (300) einstückig angeordnet sind; der Aufnahmeraum (210) auf ein von dem aktiven Biegeabschnitt entferntes Ende des passiven Biegeabschnitts aufgeschoben ist; und der Instrumentenkanal mit der ersten Öffnung (121) in Verbindung steht.

## Revendications

1. Connecteur pour une unité d'insertion d'un endoscope, comprenant :
une unité de fixation (100) et une unité de montage (200), dans lequel
un canal d'actionnement (110) est ménagé à l'intérieur de l'unité de fixation (100), et le canal d'actionnement (110) est pourvu d'une première ouverture (121) située sur une surface de l'unité de fixation (100) ;
une extrémité de l'unité de montage (200) est fixée à l'unité de fixation (100), et l'autre extrémité de l'unité de montage (200) est configurée pour être connectée à une unité d'insertion (300) ; et l'unité d'insertion (300) est pourvue d'un canal d'instrument (310), d'un canal de fil de traction (320) et d'un canal de câblage (330) ; et
lorsque l'unité de montage (200) est connectée à l'unité d'insertion (300), la première ouverture (121) est en communication avec le canal d'instrument (310), et le canal de fil de traction (320) et le canal de câblage (330) sont en communication avec un environnement externe de l'unité de fixation (100) ;
l'extrémité de l'unité de montage (200) connectée à l'unité d'insertion (300) est pourvue d'une cavité de réception (210) ; la cavité de réception (210) est en communication avec la première ouverture (121) ;
dans lequel la cavité de réception (210) est pourvue de deux extrémités ouvertes le long d'une direction axiale ; **caractérisé en ce que** : la cavité de réception (210) est emmanchée à l'extérieur de l'unité d'insertion (300), et une paroi latérale de la cavité de réception (210) est pourvue d'un premier espace (220) ; et deux extrémités du premier espace (220) sont en communication avec les deux extrémités ouvertes, respectivement.

2. Connecteur pour l'unité d'insertion de l'endoscope selon la revendication 1, dans lequel
une direction de longueur du premier espace (220) est parallèle à la direction axiale de la cavité de réception (210) ; en variante, une longueur d'arc du premier espace (220) le long d'une direction radiale de la cavité de réception (210) est définie comme une largeur du premier espace (220) ; et un angle central correspondant à la largeur du premier espace (220) est inférieur à 180°.

3. Connecteur pour l'unité d'insertion de l'endoscope selon la revendication 2, dans lequel
le premier espace (220) est divisé en un segment constant (221) et un segment d'expansion de diamètre (222) le long d'une direction allant de la cavité de réception (210) jusqu'à l'unité de fixation (100) ; une largeur du segment constant (221) reste inchangée, et une largeur du segment d'expansion de diamètre (222) augmente graduellement le long de la direction allant de la cavité de réception (210) jusqu'à l'unité de fixation (100).

4. Connecteur pour l'unité d'insertion de l'endoscope selon la revendication 1, dans lequel
une surface d'adaptation (211) est ménagée sur une paroi latérale externe de la cavité de réception (210), et la surface d'adaptation (211) est une structure plane.

5. Connecteur pour l'unité d'insertion de l'endoscope selon la revendication 1, dans lequel l'unité de fixation (100) est pourvue d'une surface d'extrémité de connexion attachée à une surface d'extrémité de l'unité d'insertion (300), et la première ouverture (121) est située sur la surface d'extrémité de connexion ; et
un bord de la surface d'extrémité de connexion est pourvu d'un premier segment incurvé (131) et d'au moins un deuxième segment incurvé (132) ; et le premier segment incurvé (131) est configuré pour être attaché à un bord du canal de câblage (330), et le deuxième segment incurvé (132) est configuré pour être attaché à un bord du canal de fil de traction (320).

6. Connecteur pour l'unité d'insertion de l'endoscope selon la revendication 1, dans lequel le canal d'actionnement (110) comprend un segment de connexion (111) et un segment d'actionnement (112) ; une extrémité du segment de connexion (111) forme la première ouverture (121), et l'autre extrémité du segment de connexion (111) forme une deuxième ouverture (122) ; et la deuxième ouverture (122) est en communication avec le segment d'actionnement (112) ; et
une surface de projection de la première ouverture (121) sur un plan où la deuxième ouverture (122) est située le long d'une direction axiale du segment de connexion (111) est située à l'intérieur de la deuxième ouverture (122).

7. Connecteur pour l'unité d'insertion de l'endoscope selon la revendication 6, dans lequel une ligne d'intersection entre une paroi latérale interne du segment de connexion (111) et un plan comprenant un axe du segment de connexion (111) est définie comme première ligne d'intersection ; la première ligne d'intersection est structurée sous la forme d'une ligne droite ou d'une ligne incurvée ; et une distance entre la première ligne d'intersection et l'axe du segment de connexion (111) le long d'une direction allant de la première ouverture (121) jusqu'à la deuxième ouverture (122) augmente graduellement ; et
une ligne d'intersection entre une paroi latérale externe du segment de connexion (111) et le plan comprenant l'axe du segment de connexion (111) est définie comme deuxième ligne d'intersection ; la deuxième ligne d'intersection est structurée sous la forme d'une ligne droite ou d'une ligne incurvée ; et une distance entre la deuxième ligne d'intersection et l'axe du segment de connexion (111) le long de la direction allant de la première ouverture (121) jusqu'à la deuxième ouverture (122) augmente graduellement.

8. Structure de fixation pour une unité d'insertion d'un endoscope, comprenant le connecteur pour l'unité d'insertion de l'endoscope selon l'une quelconque des revendications 1 à 7 et l'unité d'insertion (300), dans laquelle
une section de courbure active et une section de courbure passive de l'unité d'insertion (300) sont ménagées d'un seul tenant ; la cavité de réception (210) est emmanchée sur une extrémité de la section de courbure passive éloignée de la section de courbure active ; et le canal d'instrument est en communication avec la première ouverture (121).
